# EUROPÄISCHE PATENTSCHRIFT

(11) **EP 4 291 945 B1**
(45) Veröffentlichungstag und Bekanntmachung des Hinweises auf die Patenterteilung: **02.04.2025**
(21) Anmeldenummer: 22700982.6
(22) Anmeldetag: 21.01.2022
(51) Int. Cl.: G02C 11/08

(54) **BRILLENSCHEIBE**
SPECTACLE LENS
VERRE DE LUNETTES

(30) Priorität: 09.02.2021 DE 102021201180
(43) Veröffentlichungstag der Anmeldung: 20.12.2023
(73) Patentinhaber: Uvex Arbeitsschutz GmbH, 90766 Fürth (DE)
(72) Erfinder: JASCHKE, Simon, 90559 Burgthann (DE); WACKER, Marco, 91452 Wilhermsdorf (DE); KÜHNLEIN, Florian, 90587 Veitsbronn (DE); WOLF, Martin, 91522 Ansbach (DE)
(74) Vertreter: Rau, Schneck & Hübner Patentanwälte Rechtsanwälte PartGmbB
(86) Internationale Anmeldenummer: PCT/EP2022/051308
(87) Internationale Veröffentlichungsnummer: WO 2022/171415

(56) Entgegenhaltungen:
- EP-A1- 3 709 775
- EP-A2- 1 072 205
- WO-A1-2015/048564
- WO-A1-2017/070628
- DE-A1- 102015 114 507
- DE-U1- 202009 010 212
- US-A1- 2011 126 345
- US-A1- 2013 212 765
- US-A1- 2018 000 648
- US-A1- 2018 014 359
- US-A1- 2018 124 878

## Beschreibung

Die Erfindung betrifft eine Brillenscheibe gemäß dem Oberbegriff des Anspruchs 1. Ferner richtet sich die Erfindung auf eine Brille mit mindestens einer Brillenscheibe. Die Brille ist insbesondere als Arbeitsschutzbrille oder Wintersport-Brille, wie Skibrille, Snowboardbrille oder dergleichen, ausgeführt.

Beheizbare Brillenscheiben sind aus dem Stand der Technik allgemein bekannt. Diese haben im Betrieb oftmals einen äußerst hohen Energieverbrauch, was ein häufiges Aufladen bzw. Austauschen von Batterien erfordert. Die Brillen mit solchen Brillenscheiben sind meistens sperrig und schwer aufgrund der eingesetzten Schaltungen bzw. Leiterplatten.

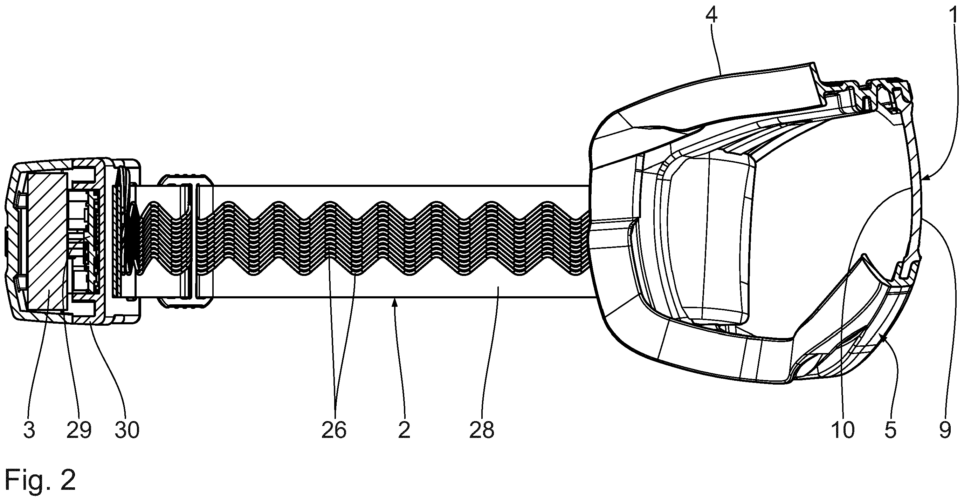

Die WO 2015/048564 A1 offenbart gattungsgemäße Antibeschlag-Augenschirme, die beispielsweise in Brillen oder für den Augenschutz einsetzbar sind. Der Augenschirm umfasst ein leitfähiges Heizelement, das in mehrere Bereiche aufgeteilt ist, um beispielsweise ein individuelles Heizen der Bereiche zu ermöglichen. Eine Vielzahl elektrisch leitfähiger Bereiche aus optisch transparentem, elektrisch-resistivem, leitfähigem Heizmaterial ist auf einem Substrat angeordnet. Der Augenschirm umfasst außerdem mindestens zwei Stromschienen, die mit den leitfähigen Bereichen verbunden und ausgebildet sind, um die leitfähigen Bereiche mit der Stromquelle zu verbinden. Gemäß Fig. 2 sind auf dem Augenschirm drei elektrisch isolierte Heizelementbereiche angeordnet, die mit einer Stromquelle in Verbindung stehen. Das Segmentieren eines Heizelements in mehrere Bereiche ermöglicht die Option, jeden Bereich separat zu beheizen, wie mit separaten Batterien oder mit PWM-Kanalsteuerungen. Die Beheizung dieser Antibeschlag-Augenschirme ist nicht immer zufriedenstellend.

Die US 2011/0126345 A1 offenbart eine Antibeschlaganordnung, wie Scheiben für den Augenschutz. Eine transparente leitende Schicht ist auf einer Fläche der Scheibe ausgebildet. Eine erste lineare Elektrode ist oben auf der Fläche der Scheibe und eine zweite lineare Elektrode ist unten auf der Fläche der Scheibe angeordnet. Die Enden der Elektroden sind mit einer Energiequelle verbunden. Jedes Ende einer Elektrode ist als Anschlussteil ausgeführt. Die transparente leitende Schicht kann mindestens in einen linken, rechten, oberen und unteren Abschnitt unterteilt sein. Die Elektroden sind dann mit den entsprechenden Bereichen verbunden.

Die DE 10 2015 114 507 A1 offenbart einen elektrisch beheizbaren Schichtkörper, dessen erster Träger ein- oder beidseitig mit nichttransparenten Leiterbahnen belegt ist. Stark gebündelte Leiterbahnen, wie Kontakte und/oder Anschlüsse, sind im Randbereich angeordnet. Nichttransparente Leiterbahnen, die eine Heizfunktion erfüllen, sind optisch transparent über einen mittigen Bereich verlegt. Die Leiterbahnen bewirken bei Anlegen elektrischen Stroms eine Beheizung des Schichtkörpers, die entweder über die Fläche hinweg gleichmäßig und/oder in einzelnen Flächenbereichen gezielt einstellbar ist. Beispielsweise kann gegenüber den Augen die Heizleistung höher eingestellt sein als beispielsweise im Nasenbereich.

Die DE 1 951 643 A offenbart eine Schutzbrille mit einer Klarsichtscheibe. Im Inneren der Klarsichtscheibe sind elektrische Heizelemente ausgebildet, die mittels elektrischer Leiter an einer in/an einer Fassung der Klarsichtscheibe beziehungsweise einem Halteband angeordneten Stromquelle anschaltbar sind. Bevorzugt können die Heizelemente und/oder Leiter durch Metallbedampfung, insbesondere von in den Augenbereichen sich erstreckenden Brillenabschnitten, erzielt sein. Sie können auch anderweitig, zum Beispiel durch Widerstandsdrähte oder gedruckte Metallschichten, gebildet sein. Die Klarsichtscheibe ist bevorzugt mit mit den Heizelementen in Verbindung stehenden Kontaktnippeln oder dergleichen ausgerüstet, an die an der Fassung oder dem Tragband festgelegte Stromquellen über elektrische Leiter lösbar ansteckbar sind. Die als Heizelemente dienenden Bedampfungsschichten, Metalldrähte oder dergleichen können sich in oder an der Klarsichtscheibe ausgebildeten Leiterplatten erstrecken. Die Stromquellen sind unmittelbar oder mittels Aufnahmebehälter an der Fassung und/oder Klarsichtscheibe beziehungsweise dem Halteband festgelegt. Diese Brille hat im Betrieb einen hohen Energieverbrauch und ist außerdem sperrig. Die DE 27 18 679 A1 offenbart eine heizbare Sichtscheibe, insbesondere für eine Skibrille, die mit einem Heizelement versehen ist. Das Heizelement ist über einen Feuchtigkeitssensor an eine Stromquelle angeschlossen. Es ist zweckmäßig, wenn das Heizelement durch eine elektrisch leitende Beschichtung gebildet ist, die auf eine Folie angebracht ist, die auf der Innenseite der Sichtscheibe befestigt ist. Auf die Beschichtung sind zwei Flächenkontakte angebracht, an die eine elektrische Spannung anlegbar ist. Diese Sichtscheibe hat sich in der Praxis bewährt.

Der Erfindung liegt die Aufgabe zugrunde, eine verbesserte Brillenscheibe und Brille zu schaffen. Insbesondere sollen die Brillenscheibe und Brille dem Träger einen hohen Komfort bieten und besonders benutzerfreundlich sein.

Diese Aufgabe wird erfindungsgemäß durch die in den Ansprüchen angegebenen Merkmale gelöst. Der Kern der Erfindung liegt in mindestens einer Beschichtung, die einen Grundkörper der Brillenscheibe be-deckt und mit elektrischen Kontaktmitteln zum elektrischen Beheizen der Brillenscheibe in elektrischer Verbindung steht. Die Kontaktmittel und die mindestens eine Beschichtung sind im Stande, elektrischen Strom bzw. elektrische Spannung zu führen.

Durch die beidseitige Anordnung von mindestens einer Leiterbahn bzw. von mindestens einem Kontaktmittel ist eine besonders gleichmäßige Aufheizung der Brillenscheibe möglich. Es ist zweckmäßig, wenn jede Leiterbahn bereichsweise auf der Brillenscheibe-Innenseite und Brillenscheibe-Außenseite der Brillenscheibe verläuft.

Die unabhängig voneinander betätigbaren Heizzonen sind bevorzugt durch die Schaltung betätigbar, wie steuerbar bzw. regelbar. Beispielsweise unterscheiden sich im Betrieb die Heizleistungen der Heizzonen voneinander. Beispielsweise ist mindestens eine Heizzone an, während mindestens eine andere Heizzone gerade aus ist. Ein Betrieb mit reduzierter oder voller Heizleistung ist möglich. Die Heizleistung von jeder Heizzone kann beispielsweise zwischen 0% und 100% liegen. Sie ist bevorzugt stufenlos einstellbar.

Eine andere bevorzugte Ausgestaltung der Brillenscheibe umfasst einen Grundkörper, mindestens eine transparente, elektrisch leitfähige Beschichtung, die den Grundkörper zumindest bereichsweise bedeckt, und elektrische Kontaktmittel, die die mindestens eine Beschichtung bereichsweise bedecken und mit der mindestens einen Beschichtung in elektrischer Verbindung stehen sowie mindestens eine elektrische Heizzone mit bilden. Die Brillenscheibe hat ferner eine Sensorplatine mit mindestens einem Federkontakt zur elektrischen Verbindung mit Leiterbahnen, wobei die Kontaktmittel mit mindestens einer elektrischen oder elektronischen Schaltung elektrisch verbunden oder verbindbar sind.

Der mindestens eine Federkontakt sorgt für eine funktionssichere elektrische Verbindung zwischen der Sensorplatine/Brillenscheibenplatine und den Leiterbahnen. Der mindestens eine Federkontakt ist insbesondere im Stande, trotz Krümmung des Grundkörpers bzw. der Brillenscheibe eine sichere elektrische Verbindung dort herzustellen. Er hat bevorzugt mindestens ein federnd verlagerbares, elektrisch leitfähiges Federkontaktmittel, wie Plättchen, Stift oder dergleichen.

Die Brillenscheibe ist beispielsweise als durchgängige Brillenscheibe, also Vollsichtscheibe, ausgebildet, die sich beim Tragen der Brille vor beiden Augen des Brillenträgers erstreckt. Alternativ ist sie als Brillenscheibe ausgebildet, die sich beim Tragen nur vor genau einem Auge des Brillenträgers erstreckt. Die Brille hat dann zwei nebeneinander angeordnete, getrennte Brillenscheiben. Die Brillenscheibe ist transparent und bevorzugt mehrschichtig. Sie ist beispielsweise getönt. Alternativ ist sie farblos. Es ist zweckmäßig, wenn mit der Brillenscheibe ein Brillenrahmen oder mindestens ein Rahmenteil in Verbindung steht. Günstigerweise ist in der Brillenscheibe und/oder benachbart zu dieser mindestens eine Belüftungsöffnung angeordnet.

Es ist von Vorteil, wenn der Grundkörper aus Glas und/oder Kunststoff, wie Polycarbonat, gebildet ist. Er ist bevorzugt durch Spritzgießen gebildet. Günstigerweise ist er ein Formkörper.

Die mindestens eine Beschichtung ist bevorzugt zusammenhängend. Alternativ ist sie beispielsweise durch voneinander getrennte Beschichtungsbereiche gebildet. Die mindestens eine Beschichtung bedeckt vorzugsweise den Grundkörper auf mindestens einer Seite günstigerweise zumindest bereichsweise, bevorzugt zumindest größtenteils, bevorzugt vollständig. Es ist zweckmäßig, wenn sich die Beschichtung innenseitig und/oder außenseitig an/auf dem Grundkörper, also an/auf der dem Brillenträger zugewandten und/oder abgewandten Seite beim Tragen der Brille, befindet. Sie steht mit dem Grundkörper in direkter oder indirekter Verbindung.

Die mindestens eine Beschichtung ist bevorzugt durch Drähte, insbesondere Nanodrähte, und/oder Partikel gebildet. Es ist zweckmäßig, wenn die mindestens eine Beschichtung elektrisch leitfähiges Metall, wie Silber, aufweist. Vorzugsweise ist die Beschichtung durch oder mit Silber-Nanodrähte(n) gebildet.

Die elektrischen Kontaktmittel sind vorzugsweise flächig und bevorzugt länglich ausgeführt. Sie sind beispielsweise streifenförmig, leistenförmig oder dergleichen. Die Kontaktmittel erstrecken sich zum Beispiel zumindest bereichsweise gerade, gekrümmt, bogenförmig oder dergleichen. Kombinationen sind möglich. Die Kontaktmittel sind bevorzugt aus einem elektrisch leitfähigen Metall gebildet und mit mindestens einer elektrischen oder elektronischen Schaltung, beispielsweise direkt oder indirekt, unter Bildung eines Stromkreises elektrisch verbunden oder verbindbar. Es ist zweckmäßig, wenn sie mit mindestens einer Energiequelle bzw. Stromquelle, wie Batterie, Akku oder dergleichen, direkt oder indirekt, elektrisch verbunden bzw. verbindbar sind. Der Akku ist bevorzugt kabellos/berührungslos elektrisch aufladbar.

Jede elektrische Heizzone der Brillenscheibe bedeckt den Grundkörper. Sie ist durch die zugehörigen Kontaktmittel und günstigerweise die mindestens eine Beschichtung gebildet. Jede elektrische Heizzone ist vorzugsweise durch mindestens zwei elektrische Kontaktmittel mit gebildet, die bevorzugt einander gegenüberliegend, günstigerweise in unterschiedlichen Höhen der Brillenscheibe, wie unten und oben, angeordnet sind. Durch unterschiedliche Heizzonen ist eine homogene bzw. gezielte Aufheizung der Brillenscheibe möglich. Es ist zweckmäßig, wenn Heizzyklen situationsabhängig und/oder zeitgesteuert erfolgen.

Die elektrischen Leiterbahnen sind bevorzugt länglich ausgeführt und verlaufen günstigerweise bereichsweise parallel zueinander. Sie sind aus einem elektrisch leitfähigen Metall gebildet und im Stande, elektrischen Strom bzw. elektrische Spannung zu führen. Die Leiterbahnen sind mit mindestens einer elektrischen oder elektronischen Schaltung, beispielsweise direkt oder indirekt, unter Bildung eines Stromkreises elektrisch verbunden oder verbindbar. Es ist zweckmäßig, wenn sie mit mindestens einer Energiequelle bzw. Stromquelle, wie Batterie, Akku oder dergleichen, direkt oder indirekt, elektrisch verbunden bzw. verbindbar sind.

Es ist zweckmäßig, wenn mindestens eine elektrische/elektronische Komponente austauschbar ist.

Günstigerweise hat die Brille zum Tragen derselben mindestens ein Kopf-Haltemittel zum Halten der mindestens einen Brillenscheibe an dem Kopf des Brillenträgers. Das mindestens eine Kopf-Haltemittel ist beispielsweise als Kopfband, Brillenbügel oder dergleichen ausgeführt. Es ist von Vorteil, wenn das Kopfband ein Längenverstellmittel umfasst. Es ist zweckmäßig, wenn die elektrische oder elektronische Schaltung beabstandet zu der Brillenscheibe, beispielsweise an dem Kopf-Haltemittel der Brille und bevorzugt gegenüberliegend zu der Brillenscheibe, angeordnet ist. Dies sorgt für eine besonders gute Gewichtsverteilung und somit einen äußerst hohen Tragekomfort.

Weitere vorteilhafte Ausgestaltungen der Erfindung sind in den Unteransprüchen angegeben.

Die Heizzonen gemäß dem Unteranspruch 2 sind beispielsweise getrennt voneinander. Alternativ schließen sich diese direkt aneinander an. Es ist zweckmäßig, wenn zwischen zwei und fünf, bevorzugt zwei oder drei, Heizzonen vorhanden sind.

Die Leiterbahnen und/oder Kontaktmittel gemäß dem Unteranspruch 3 sind besonders kostengünstig und funktionssicher herstellbar. Sie haben nur einen geringen Platzbedarf.

Die Antibeschlag-Überbeschichtung gemäß dem Unteranspruch 4 ist bevorzugt durchgängig. Alternativ ist sie durch einzelne Bereiche gebildet. Die Antibeschlag-Überbeschichtung verhindert bzw. verlangsamt einen Beschlag der Brillenscheibe, wenn die mindestens eine Heizzone gerade inaktiv ist, beispielsweise aufgrund einer leeren Stromquelle. Es ist zweckmäßig, wenn die Antibeschlag-Überbeschichtung die mindestens eine Beschichtung und bevorzugt die Kontaktmittel zumindest bereichsweise bedeckt. Sie hat eine freie Seite, die beim Tragen der Brille dem Brillenträger zugewandt ist. Es ist zweckmäßig, wenn die Antibeschlag-Überbeschichtung eine nasschemische Beschichtung ist. Sie steht bevorzugt mit dem Grundkörper in indirekter Verbindung. Es ist von Vorteil, wenn sie mit der mindestens einen Beschichtung in direkter Verbindung steht.

Es ist zweckmäßig, wenn die Brillenscheibe mindestens einen Sensor für eine Strombeaufschlagung bei Sensieren einer Abweichung von einem Sollwert oder Sollwertbereich aufweist. Der mindestens eine Sensor ermöglicht eine gezielte, bedarfsabhängige Strombeaufschlagung der Kontaktmittel. Er ist beispielsweise im Stande, eine Sättigung der Antibeschlag-Überbeschichtung, eine vorherrschende Temperatur der Brillenscheibe und/oder deren benachbarten Umgebung und/oder eine vorherrschende Feuchte der Brillenscheibe und/oder deren benachbarten Umgebung zu sensieren. Es ist zweckmäßig, wenn der mindestens eine Sensor direkt an der Brillenscheibe angeordnet ist. Alternativ ist dieser beabstandet, aber benachbart zu der Brillenscheibe angeordnet. Der mindestens eine Sensor ist bevorzugt mit der Schaltung, beispielsweise direkt oder indirekt, elektrisch verbunden oder verbindbar. Er ist im Stande, die sensierte/n Größe/Größen in ein entsprechendes, insbesondere weiterverarbeitbares, elektrisches Signal umzuformen.

Die mindestens eine harte Schicht gemäß dem Unteranspruch 5 führt zu einer besonders kratzfesten Brillenscheibe. Beschädigungen an der Brillenscheibe sind so vermeidbar bzw. reduzierbar. Die mindestens eine harte Schicht steht mit dem Grundkörper in direkter oder indirekter Verbindung. Sie steht mit der mindestens einen Beschichtung in direkter oder indirekter Verbindung. Sie ist innenseitig und/oder außenseitig in Bezug auf den Grundkörper angeordnet. Jede harte Schicht hat eine freie Seite, die beim Tragen der Brille dem Brillenträger zugewandt oder abgewandt ist.

Der Grundkörper gemäß dem Unteranspruch 6 ist zumindest bereichsweise, wie zweidimensional oder dreidimensional, bevorzugt stark, gekrümmt. Es ist zweckmäßig, wenn die mindestens eine Beschichtung, Antibeschlag-Überbeschichtung, sofern vorhanden, harte Schicht, sofern vorhanden, und mindestens eine Heizzone der Krümmung folgt.

Es ist von Vorteil, wenn die Brillenscheibe mit mindestens einer Elektronikkomponente und/oder Sensorkomponente direkt bestückt ist. Die Elektronikkomponente ist beispielsweise Bestandteil der Schaltung. Alternativ bildet diese die Schaltung vollständig. Die Elektronikkomponente ist zum Beispiel als Microcontroller ausgeführt. Die Sensorkomponente ist beispielsweise Bestandteil des mindestens einen Sensors. Alternativ bildet diese den mindestens einen Sensor vollständig.

Es ist von Vorteil, wenn die Brille ein Kopfband zum Halten der Brille an einem Kopf eines Brillenträgers umfasst, wobei das Kopfband aufweist einen biegsamen, elastischen, länglichen Tragkörper und elektrische Verbindungsleitungen zur Verbindung einer Stromquelle mit elektrischen Kontaktmitteln, wobei die Verbindungsleitungen an dem Tragkörper zumindest bereichsweise derart angeordnet sind, dass diese im Stande sind, ihre Länge in einer Längsrichtung des Tragkörpers zu verändern. Die Verbindungsleitungen erlauben eine Größenanpassung des Kopfbands an den Brillenträger, was besonders benutzerfreundlich ist. Es ist zweckmäßig, wenn die Verbindungsleitungen einen Flachbandleiter bilden und bevorzugt zumindest bereichsweise parallel nebeneineinander, wie mäanderartig, schlangenlinienförmig, zickzackförmig oder dergleichen, verlaufen. Sie können zumindest bereichsweise beabstandet zueinander verlaufen oder aneinander anliegen. Sie sind günstigerweise zumindest längs ihrer Längserstreckung an sich undehnbar und bevorzugt elektrisch isoliert.

Günstigerweise sind die Verbindungsleitungen bereichsweise an dem Tragkörper fixiert.

Nachfolgend werden unter Bezugnahme auf die beigefügte Zeichnung bevorzugte Ausführungsformen der Erfindung beispielhaft beschrieben. Dabei zeigen:
- Fig. 1: eine perspektivische Ansicht einer erfindungsgemäßen Brille mit einer Brillenscheibe der Erfindung,
- Fig. 2: einen Schnitt durch die in Figur 1 veranschaulichte Brille längs einer ersten Hauptebene der Brille,
- Fig. 3: einen Schnitt durch in Figur 1 dargestellte Brille längs einer Ebene, die senkrecht zu der Hauptebene gemäß Figur 2 verläuft,
- Fig. 4: eine perspektivische Ansicht, die isoliert elektrische bzw. elektronische Komponenten der in Figuren 1 bis 3 dargestellten Brille zeigt,
- Fig. 5: ein vereinfachtes Blockschaltbild, das die Wechselwirkung der elektrischen bzw. elektronischen Komponenten der in Figuren 1 bis 3 gezeigten Brille veranschaulicht,
- Fig. 6: eine Ansicht von außen auf die Brillenscheibe der in Figuren 1 bis 3 gezeigten Brille,
- Fig. 7: eine Ansicht von innen auf die Brillenscheibe der in Figuren 1 bis 3 veranschaulichten Brille,
- Fig. 8: einen Seitenbereich der Brillenscheibe der in Figuren 1 bis 3 gezeigten Brille,
- Fig. 9: einen Schnitt durch die Brillenscheibe der in Figuren 1 bis 3 veranschaulichten Brille,
- Fig. 10: das in Figur 9 gekennzeichnete Detail X,
- Fig. 11: eine Seitenansicht der in Figuren 1 bis 3 dargestellten Brillescheibe, die auch eine Sensorplatine zeigt,
- Fig. 12: eine Schnittansicht, die einen möglichen Aufbau der Brillenscheibe der in Figuren 1 bis 3 dargestellten Brille veranschaulicht,
- Fig. 13 bis 15: Schnittansichten, die jeweils einen alternativen Aufbau der Brillenscheibe der in Figuren 1 bis 3 dargestellten Brille zeigen, und
- Fig. 16: einen Seitenbereich einer alternativen Brillenscheibe der in Figuren 1 bis 3 dargestellten Brille

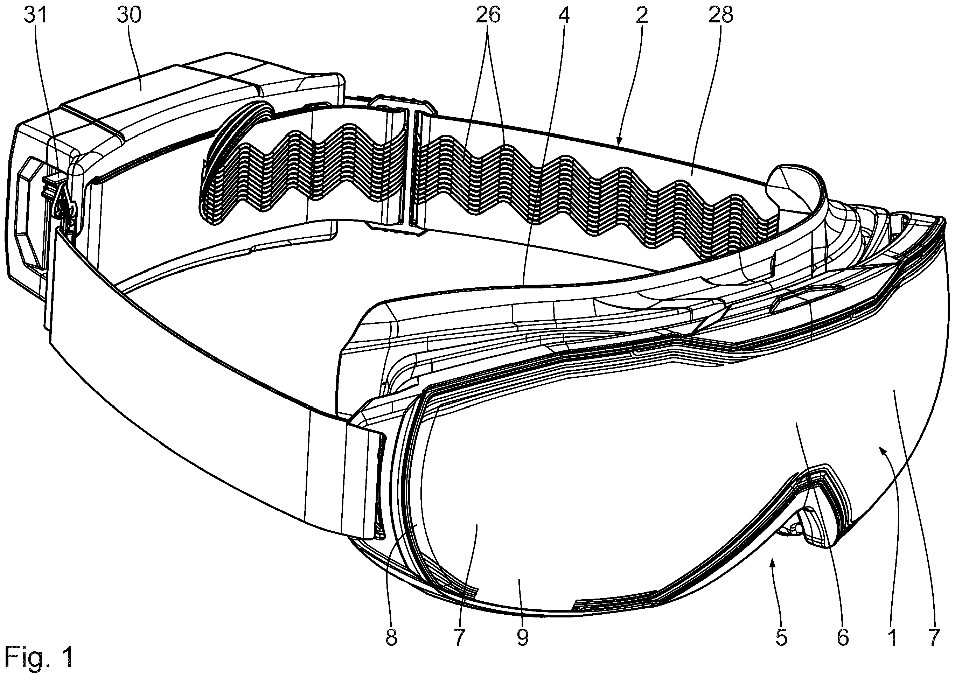

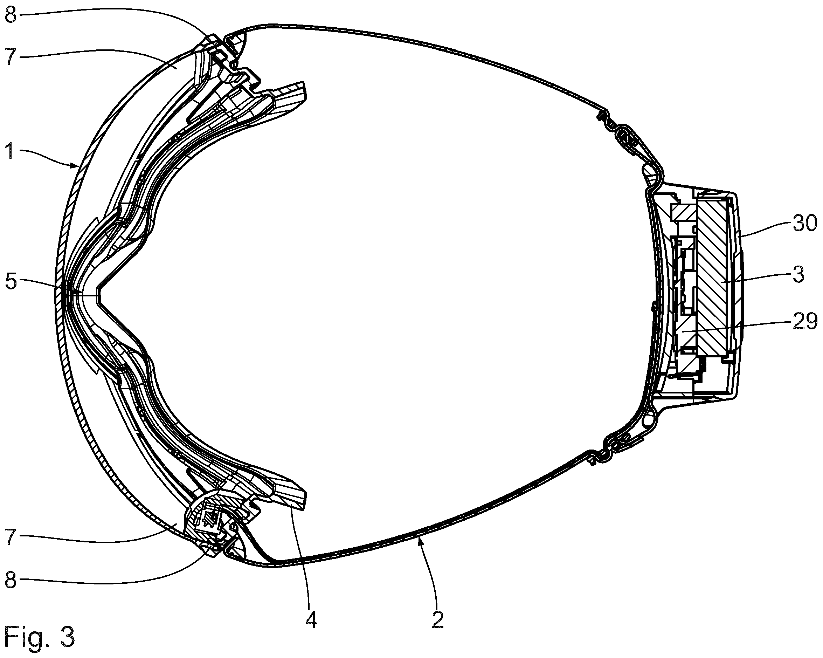

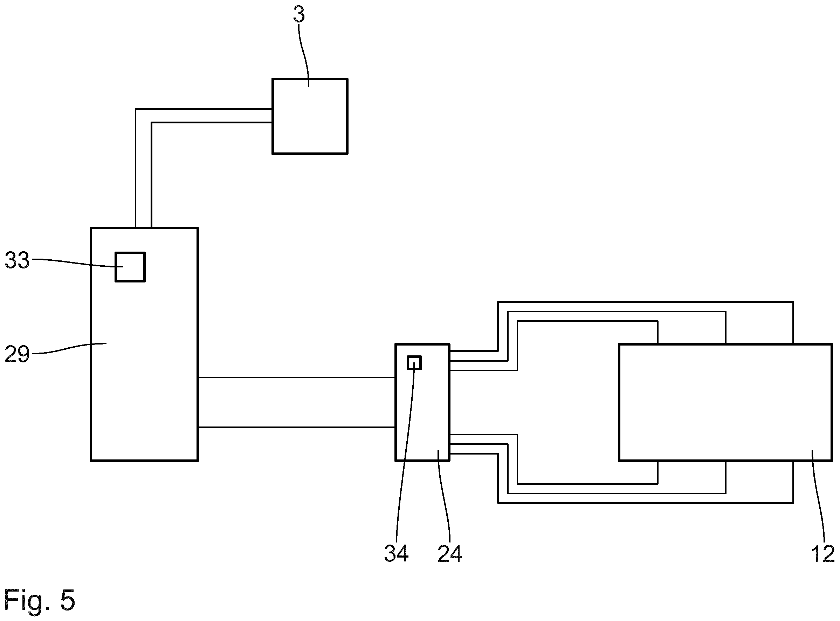

Einander entsprechende Teile sind in den Figuren 1 bis 16 mit denselben Bezugszeichen versehen. Auch Einzelheiten der im Folgenden näher erläuterten bevorzugten Ausführungsformen können für sich genommen eine Erfindung darstellen oder Teil eines Erfindungsgegenstands sein.

Eine in Figuren 1 bis 3 in ihrer Gesamtheit dargestellte Brille umfasst eine elektrisch beheizbare Vollsicht-Brillenscheibe 1 und ein mit der Brillenscheibe 1 in Verbindung stehendes Kopfband 2 sowie eine an dem Kopfband 2 angeordnete Stromquelle 3 für ein elektrisches Beheizen der Brillenscheibe 1.

An der Brillenscheibe 1 ist randseitig ein Dichtkörper 4 zur Anlage an einem Gesicht eines Brillenträgers (nicht dargestellt) angeordnet. Der Dichtkörper 4 ist länglich und bevorzugt in Umfangsrichtung geschlossen. Er ist nachgiebig und beispielsweise aus Schaumstoff, Gummi oder dergleichen ausgeführt. Der Dichtkörper 4 begrenzt räumlich mit der Brillenscheibe 1 einen Innenraum.

Die Brillenscheibe 1 weist über einer zentralen Nasenaussparung 5 einen zentralen Bereich 6 und seitlich zu diesem jeweils einen Seitenbereich 7 auf, der sich jeweils bis zu einem Seitenrand 8 der Brillenscheibe 1 erstreckt. Ferner hat die Brillenscheibe 1 eine in getragenem Zustand der Brille dem Brillenträger abgewandte Außenseite 9 und eine diesem dann zugewandte Innenseite 10. Die Brillenscheibe 1 ist stark gekrümmt.

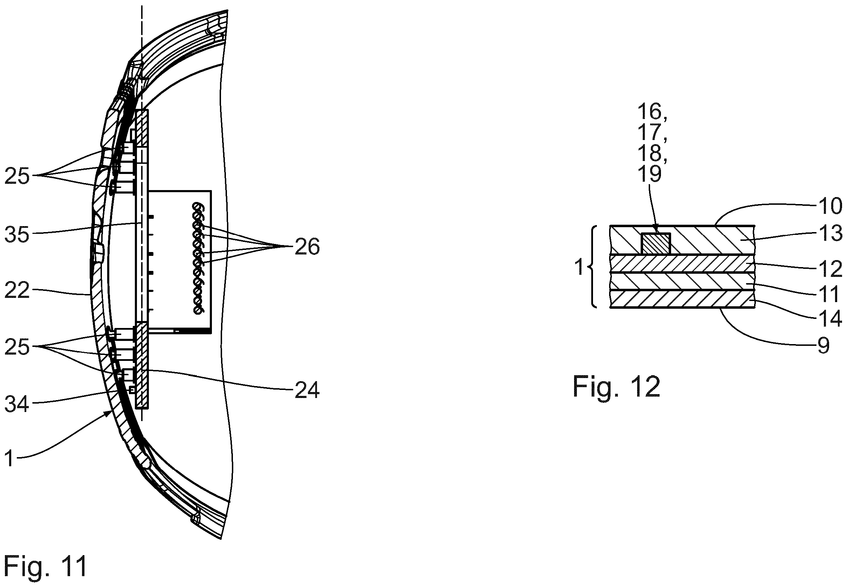

Die Brillenscheibe 1 umfasst einen Brillenscheiben-Grundkörper 11 und eine auf diesem direkt angeordnete, transparente, elektrisch leitfähige Beschichtung 12 sowie eine direkt über dieser angeordnete Antibeschlag-Überbeschichtung 13. Die Beschichtung 12 ist durch eine Vielzahl von Silber-Nanodrähten (AgNW) gebildet. Gegenüberliegend zu der Beschichtung 12 ist vorzugsweise direkt auf dem Grundkörper 11 eine harte bzw. kratzfeste Schicht 14 angeordnet. Die harte Schicht 14 bildet die Außenseite 9 aus und ist somit als Außenschicht ausgeführt. Die Antibeschlag-Überbeschichtung 13 bildet dagegen die Innenseite 10 aus und ist als Innenschicht ausgeführt (siehe Fig. 12).

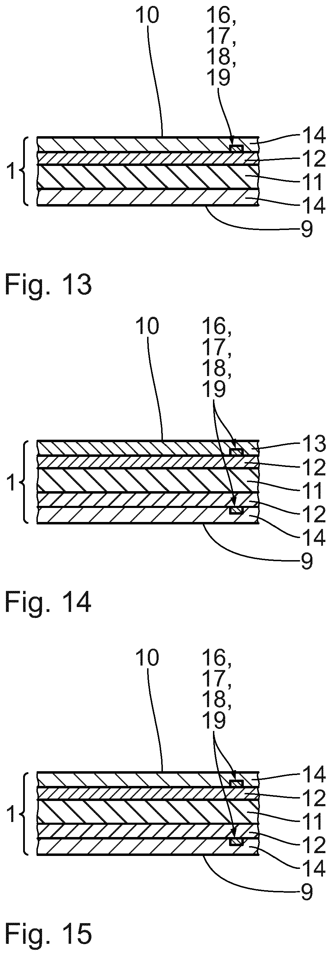

Gemäß einer alternativen Ausführungsform ist anstelle der Antibeschlag-Überbeschichtung 13 eine weitere/zweite harte Schicht 14 vorhanden (Fig. 13).

Gemäß einer alternativen Ausführungsform ist beidseits des Grundkörpers 11 jeweils direkt eine transparente, elektrisch leitfähige Beschichtung 12 angeordnet. Eine erste Beschichtung 12 ist direkt von einer die Außenseite 9 ausbildenden, harten Schicht 14 bedeckt, während die andere, zweite Beschichtung 12 direkt von einer die Innenseite 10 ausbildenden Antibeschlag-Überbeschichtung 13 bedeckt ist (Fig. 14).

Alternativ sind beide transparenten, elektrisch leitfähigen Beschichtungen 12 jeweils direkt von einer harten Schicht 14 bedeckt (Fig. 15). Eine erste harte Schicht 14 bildet die Außenseite 9 aus, während die andere/zweite harte Schicht 14 die Innenseite 10 ausbildet.

Kombinationen bzw. ein anderer Schichtaufbau ist/sind alternativ möglich.

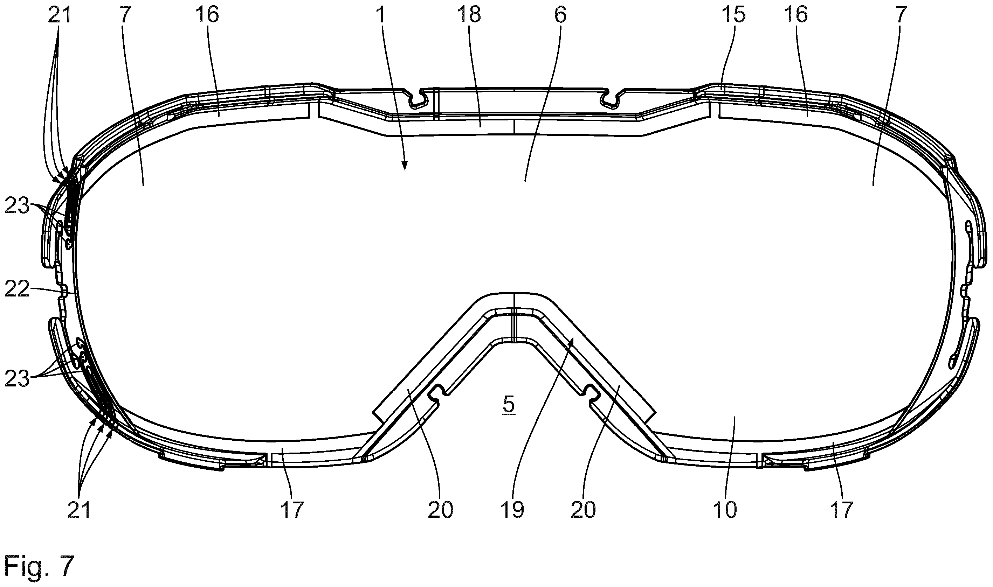

Wie beispielsweise Figur 7 zeigt, hat die Brillenscheibe 1 mehrere, längliche, elektrische Kontaktmittel, die sich benachbart bzw. längs eines Umfangsrands 15 der Brillenscheibe 1 in Umfangsrichtung der Brillenscheibe 1 erstrecken. In jedem Seitenbereich 7 sind ein oberes, seitliches Kontaktmittel 16 und ein unteres, seitliches Kontaktmittel 17 angeordnet. In dem zentralen Bereich 6 sind ein oberes, zentrales Kontaktmittel 18 und ein unteres, zentrales Kontaktmittel 19 angeordnet. Das untere, zentrale Kontaktmittel 19 folgt der Nasenaussparung 5 und hat zwei schräg zueinander verlaufende Schenkel 20.

Die Kontaktmittel 16, 17, 18, 19 sind in Umfangsrichtung beabstandet zueinander angeordnet. Die oberen, seitlichen Kontaktmittel 16 und das obere, zentrale Kontaktmittel 18 verlaufen (im Wesentlichen) in einer gemeinsamen Höhe, während die unteren, seitlichen Kontaktmittel 17 in einer gemeinsamen Höhe verlaufen. Die oberen, seitlichen Kontaktmittel 16 verlaufen oberhalb der unteren, seitlichen Kontaktmittel 17, das heißt in einer größeren Höhe als die unteren, seitlichen Kontaktmittel 17. Sie sind seitlich bzw. in einer Seitenrichtung der Brillenscheibe 1 (im Wesentlichen) unversetzt zueinander angeordnet. Das obere, zentrale Kontaktmittel 18 verläuft oberhalb des unteren, zentralen Kontaktmittels 19, das heißt in einer größeren Höhe als das untere, zentrale Kontaktmittel 19. Sie sind seitlich bzw. in einer Seitenrichtung der Brillenscheibe 1 (im Wesentlichen) unversetzt zueinander angeordnet. Die in jedem Bereich 6, 7 angeordneten Kontaktmittel 16, 17, 18 bzw. 19 bilden jeweils ein elektrisches Kontaktmittel-Paar. Es sind drei Kontaktmittel-Paare vorhanden. Die Kontaktmittel 16, 17, 18 bzw. 19 stehen mit der Beschichtung 12 in direkter elektrischer Verbindung und sind bevorzugt von der Antibeschlag-Überbeschichtung 13 bedeckt bzw. in diese eingebettet. Sie sind insbesondere zumindest zu einem gewissen Teil und insbesondere randseitig auf die Beschichtung 12 aufgedruckt.

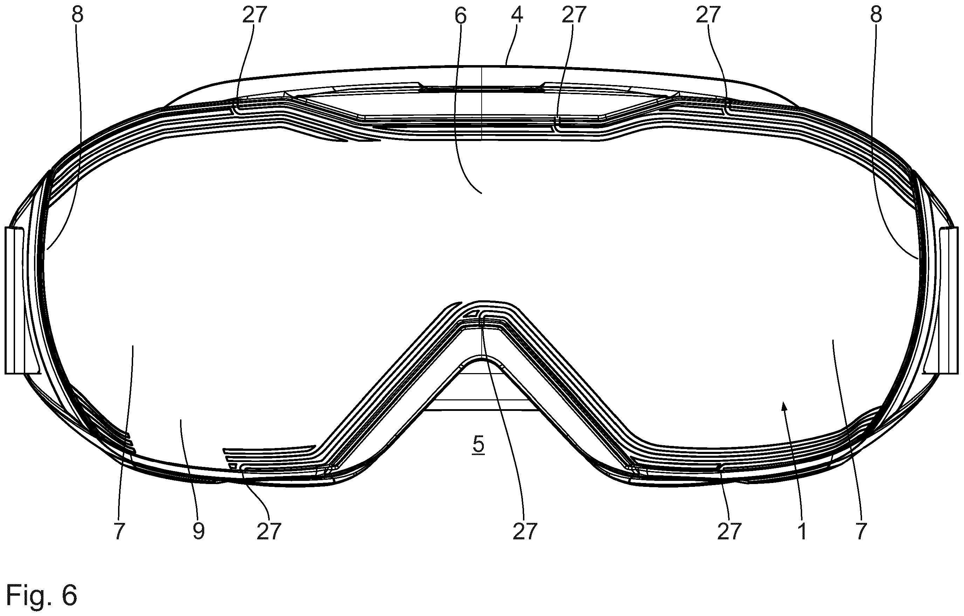

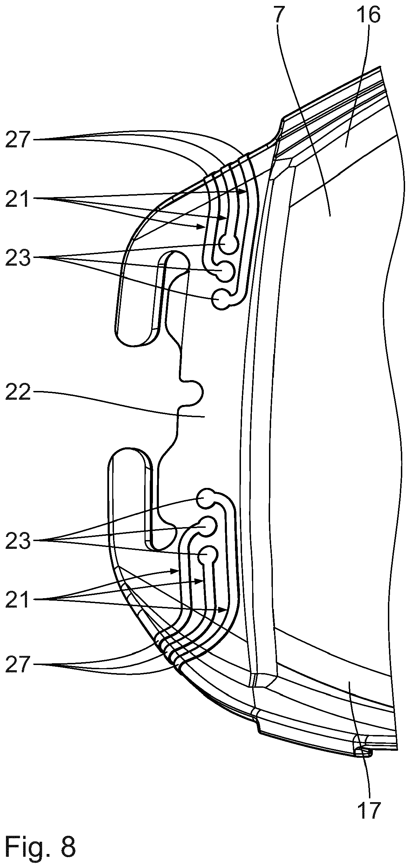

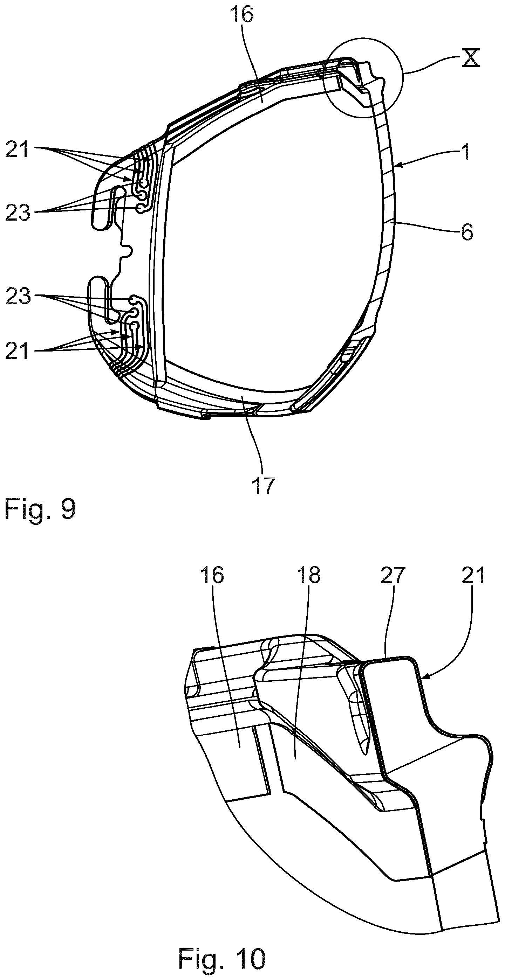

Jedes Kontaktmittel 16, 17, 18 bzw. 19 steht in elektrischer Verbindung mit einer eigenen, elektrischen Leiterbahn 21, die sich längs des Umfangsrands 15 erstreckt und in einem Seitenendbereich 22 der Brillenscheibe 1 unter Bildung einer vergrößerten, flächigen, freiliegenden Kontaktstelle 23 endet (Fig. 7, 8). Jede Leiterbahn 21 verläuft innenseitig und außenseitig in Bezug auf den Grundkörper 11 bzw. die Brillenscheibe 1. Figur 6 zeigt entsprechende Seitenwechsel 27 der Leiterbahnen 21 zwischen der Innenseite 10 und der Außenseite 9 der Brillenscheibe 1. Bei jedem Seitenwechsel 27 läuft die jeweilige Leiterbahn 21 außen um den Grundkörper 11 bzw. dessen Außenrand herum (Fig. 9, 10).

Die Kontaktmittel 16, 17, 18 bzw. 19 stehen bei der Ausführungsform gemäß Figur 13 wieder mit der Beschichtung 12 in direkter elektrischer Verbindung und sind von der weiteren, also die Innenseite 10 ausbildenden, harten Schicht 14 bedeckt.

Erste Kontaktmittel 16, 17, 18 bzw. 19 stehen bei der Ausführungsform gemäß Figur 14 mit der ersten Beschichtung 12 in direkter elektrischer Verbindung und sind von der harten Schicht 14 bedeckt. Zweite Kontaktmittel 16, 17, 18 bzw. 19 stehen mit der zweiten Beschichtung 12 in direkter elektrischer Verbindung und sind von der Antibeschlag-Überbeschichtung 13 bedeckt.

Erste Kontaktmittel 16, 17, 18 bzw. 19 stehen bei der Ausführungsform gemäß Figur 15 mit einer ersten Beschichtung 12 in direkter elektrischer Verbindung und sind von der ersten harten Schicht 14 bedeckt. Zweite Kontaktmittel 16, 17, 18 bzw. 19 stehen mit einer zweiten Beschichtung 12 in direkter elektrischer Verbindung und sind von der zweiten harten Schicht 14 bedeckt.

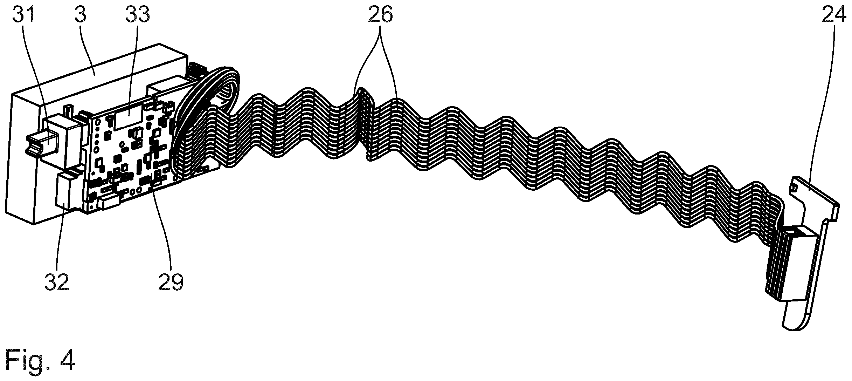

Die Brillenscheibe 1 umfasst/trägt außerdem eine Sensor-/Brillenscheibenplatine 24 (Fig. 4, 11). Die Sensorplatine 24 ist in dem Seitenendbereich 22 innenseitig angeordnet und weist stiftartige Federkontakte 25, mindestens einen Sensor 34 und bevorzugt einen eigenen Microcontroller auf. Die Federkontakte 25 sind identisch ausgeführt und in ihrer Länge federnd veränderlich. Jeder Federkontakt 25 springt von einer Hauptebene 35 der Sensorplatine 24 senkrecht vor und steht mit einer Kontaktstelle 23 einer jeweiligen Leiterbahn 21 in elektrischer Verbindung. Aufgrund der Krümmung der Brillenscheibe 1 in dem Seitenendbereich 22 haben die Federkontakte 25 unterschiedliche Längen; insbesondere nimmt die Länge von oberen Federkontakten 25 von oben nach unten zu und die Länge von unteren Federkontakten 25 von unten nach oben zu. Die Sensorplatine 24 ist bevorzugt von einer Abdeckung abgedeckt.

Jeder Federkontakt 25 steht in elektrischer Verbindung mit zwei elektrischen Verbindungsleitungen 26 (Fig. 1, 2, 4, 11), die an die Sensorplatine 24 elektrisch angeschlossen sind. Die Verbindungsleitungen 26 verlaufen von der Sensorplatine 24 über das Kopfband 2 zu der Stromquelle 3. Sie sind innenseitig an einem Tragkörper 28 des Kopfbands 2 angeordnet und sind in einer Quer- bzw. Breitenrichtung des Kopfbands 2 nebeneinander angeordnet. Die Verbindungsleitungen 26 verlaufen parallel zueinander. Sie sind mäanderförmig angeordnet und so im Stande, eine Längenveränderung des Kopfbands 2, wie Längenzunahme oder Längenabnahme, mitzumachen.

Die Verbindungsleitungen 26 sind an dem Tragkörper 28 lokal, insbesondere mittels, bevorzugt transparenten, Haltefäden, fixiert. Die Haltefäden bestehen beispielsweise aus Nylon. Sie sind vorzugsweise mit dem Tragkörper 28 vernäht und umlaufen jeweils die Verbindungsleitungen 26. Die eine Verbindungsleitung 26 fixierenden Haltefäden sind längs dieser Verbindungsleitung 26 beabstandet zueinander angeordnet.

Jede Verbindungsleitung 26 hat bevorzugt einen Durchmesser, der zwischen 0,1 mm und 0,5 mm (ohne Isolierung) und zwischen 0,5 mm und 0,8 mm (mit Isolierung) liegt. In ungedehntem Zustand des Kopfbands 2 liegt der Abstand zwischen zwei benachbarten Extremstellen, also einem Minimum und einem Maximum, der Verbindungsleitungen 26 günstigerweise zwischen 5 mm und 15 mm.

Die Verbindungsleitungen 26 sind an eine Hauptplatine 29 elektrisch angeschlossen (Fig. 2, 4), die benachbart zu der Stromquelle 3 angeordnet ist. Die Hauptplatine 29 und die Stromquelle 3 sind gemeinsam in einem Gehäuse 30 angeordnet. Das Gehäuse 30 trägt einen Ein-/Aussschalter 31, der manuell betätigbar ist und in einer Einstellung eine Stromverbindung zwischen der Stromquelle 3 und der Hauptplatine 29 erlaubt. In einer Ausstellung unterbricht der Ein-/Aussschalter 31 die Stromverbindung zwischen diesen. Ferner trägt das Gehäuse 30 eine Ladebuchse 32, die beispielsweise als USB-Buchse, insbesondere USB-C-Buchse, ausgeführt ist. Die Hauptplatine 29 wiederum trägt einen Microcontroller 33 zur Verarbeitung elektrischer Sensordaten bzw. -signale des mindestens einen Sensors 34 und zur Strombeaufschlagung der Kontaktmittel 16, 17, 18 bzw. 19.

Nachfolgend wird der Einsatz der Brille näher beschrieben.

Die Brillenscheibe 1 erstreckt sich in getragenem Zustand der Brille vor den Augen des Brillenträgers und läuft bereichsweise um dessen Gesicht. Die Nase des Brillenträgers greift in die Nasenaussparung 5 ein. Das Gehäuse 30 befindet sich im Hinterkopfbereich des Brillenträgers gegenüberliegend zu der Brillenscheibe 1.

In der Einstellung des Ein-/Ausschalters 31 wird der Microcontroller 33 mit Strom von der Stromquelle 3 beaufschlagt. Der Microcontroller 33 empfängt von dem mindestens einen Sensor 34 entsprechende elektrische Sensorsignale über die Verbindungsleitungen 26. Wenn der Microcontroller 33 feststellt, dass eine zumindest teilweise Beheizung der Brillenscheibe 1 notwendig ist, wird über die jeweilige Verbindungsleitung 26 elektrischer Strom aus der Stromquelle 3 zu der Sensorplatine 24 geführt, die über den jeweiligen Federkontakt 25 die zugehörige Leiterbahn 21 und somit das zugeordnete Kontaktmittel 16, 17, 18 bzw. 19 mit Strom beaufschlagt. Der elektrische Strom gelangt so über das jeweilige Kontaktmittel 16, 17, 18 bzw. 19 zu der Beschichtung 12. Er fließt über die Beschichtung 12 unter Bildung einer jeweiligen elektrischen Heizzone zu dem zugeordneten gegenüberliegenden Kontaktmittel 16, 17, 18 bzw. 19, von wo er über die jeweilige Leiterbahn 21 und dem zugehörigen Federkontakt 25 sowie die entsprechende Verbindungsleitung 26 zurück zu der Hauptplatine 29 fließt.

Die Heizleistung von jeder Heizzone wird bei der Ausführungsform gemäß Figur 12 insbesondere so eingestellt, dass die Antibeschlag-Überbeschichtung 13 bei maximaler Sättigung von innen beheizt und so die Feuchtigkeit wieder aus dieser ausgetrieben wird. Eine Sättigung der Antibeschlag-Überbeschichtung 13 bleibt so aus, was ein Beschlagen derselben dauerhaft verhindert und die Heizleistung auf ein Minimum reduziert. Der Microcontroller 33 der Hauptplatine 29 stellt entsprechend den Heizstrom ein, um die Antibeschlag-Überbeschichtung 13 zu entfeuchten. Eine Stromzufuhr zu den Heizzonen erfolgt demnach nur bei Bedarf. Es ist zweckmäßig, wenn der Microcontroller 33 der Hauptplatine 29 einen Auswertealgorithmus zur Auswertung der Sensorsignale aufweist/nutzt. Die Feuchtigkeit bzw. Sättigung der Antibeschlag-Überbeschichtung 13 bzw. Brillenscheibe 1 wird entsprechend durch den mindestens einen Sensor 34 sensiert. Die Stromquelle 3 versorgt die Heizzonen und die Hauptplatine 29 mit elektrischem Strom im Betrieb.

Bei den Ausführungsformen gemäß Figuren 13 und 15 wird bei dem Beheizen die Kondensation von Feuchtigkeit auf der Innenseite 10 verhindert, die jeweils an der harten Schicht 14 ausgebildet ist. Die harten Schichten 14 werden jeweils von innen beheizt. Das Beheizen erfolgt ansonsten bevorzugt gemäß der Ausführungsform gemäß Figur 12, auf deren Beschreibung verwiesen wird.

Bei den Ausführungsformen gemäß Figuren 14, 15 wird der Grundkörper 11 beidseitig erwärmt. Bei der Ausführungsform gemäß Figur 14 werden sowohl die Antibeschlag-Überbeschichtung 13 als auch die harte Schicht 14 von innen beheizt. Bei der Ausführungsform gemäß Figur 15 werden beide harte Schichten 14 von innen beheizt.

Bei der Ausführungsform gemäß Figur 14 wird die Heizleistung von jeder Heizzone insbesondere so eingestellt, dass die Antibeschlag-Überbeschichtung 13 bei maximaler Sättigung von innen beheizt und so die Feuchtigkeit wieder aus dieser ausgetrieben wird. Eine Sättigung der Antibeschlag-Überbeschichtung 13 bleibt so aus, was ein Beschlagen derselben dauerhaft verhindert und die Heizleistung auf ein Minimum reduziert. Das Beheizen erfolgt ansonsten bevorzugt gemäß der Ausführungsform gemäß Figur 12, auf deren Beschreibung verwiesen wird.

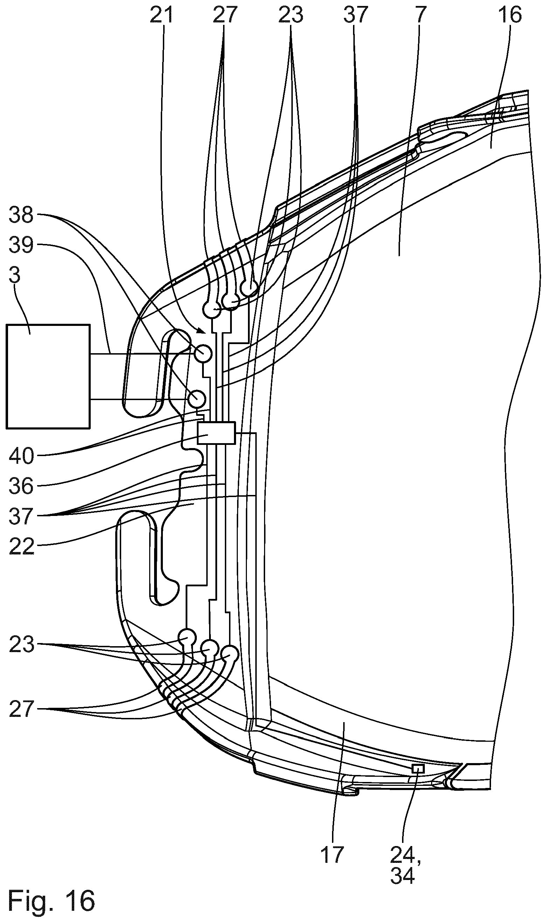

Nachfolgend wird unter Bezugnahme auf Figur 16 eine alternative Ausbildung der Brillenscheibe 1 beschrieben. Im Vergleich mit der Brillenscheibe 1 gemäß Figuren 8, 11, auf deren Beschreibung verwiesen wird, ist die Brillenscheibe 1 gemäß dieser Ausführungsform direkt mit dem mindestens einen Sensor 34 bestückt, der in einem Randbereich der Brillenscheibe 1, wie seitlich unten, angeordnet ist. Ferner ist die Brillenscheibe 1 direkt mit einem Microcontroller 36 bestückt, der in dem Seitenendbereich 22 der Brillenscheibe 1 angeordnet ist. Der Microcontroller 36 bildet beispielsweise den Microcontroller der Sensorplatine 24 und/oder den Microcontroller 33 der Hauptplatine 29.

Der mindestens eine Sensor 34 und die Kontaktstellen 23 stehen über elektrische Anschlussleitungen 37 mit dem Microcontroller 36 in elektrischer Verbindung. Ferner sind in dem Seitenbereich 22 zwei elektrische Anschlussstellen 38 angeordnet, die mit der Stromquelle 3 über elektrische Leitungen 39 verbunden bzw. verbindbar und mit dem Microcontroller 36 über elektrische Leitungen 40 elektrisch verbunden sind. Die Anschlussleitungen 37 und Leitungen 40 sind ebenfalls direkt auf die Brillenscheibe 1 aufgebracht.

Im Betrieb wird der Microcontroller 36 mit Strom von der Stromquelle 3 beaufschlagt. Der Microcontroller 36 empfängt von dem mindestens einen Sensor 34 entsprechende elektrische Signale über die zugehörige Anschlussleitung 37. Wenn der Microcontroller 36 feststellt, dass eine zumindest teilweise Beheizung der Brillenscheibe 1 notwendig ist, wird über die jeweilige Anschlussleitung 37 elektrischer Strom über die zugehörige Leiterbahn 21 zu dem zugeordneten Kontaktmittel 16, 17, 18 bzw. 19 geführt, von wo der elektrische Strom zu der Beschichtung 12 gelangt. Er fließt über die Beschichtung 12 unter Bildung einer jeweiligen elektrischen Heizzone zu dem zugeordneten gegenüberliegenden Kontaktmittel 16, 17, 18 bzw. 19, von wo er über die jeweilige Leiterbahn 21 und die entsprechende Anschlussleitung 37 zurück zu dem Microcontroller 36 fließt. Die Beheizung über die Heizzonen erfolgt im Wesentlichen analog wie bei der entsprechenden vorherigen Ausführungsform, auf die verwiesen wird.

## Patentansprüche

1. Brillenscheibe
a) mit einem Grundkörper (11),
b) mit mindestens einer transparenten, elektrisch leitfähigen Beschichtung (12), die den Grundkörper (11) zumindest bereichsweise bedeckt,
c) mit elektrischen Kontaktmitteln (16, 17, 18, 19), die
i) die mindestens eine Beschichtung (12) bereichsweise bedecken,
ii) mit der mindestens einen Beschichtung (12) in elektrischer Verbindung stehen, und
iii) mindestens zwei elektrische Heizzonen mit bilden, die unabhängig voneinander betätigbar sind, und
d) mit elektrischen Leiterbahnen (21), die mit den Kontaktmitteln (16, 17, 18, 19) zur Strombeaufschlagung derselben elektrisch verbunden sind,
**dadurch gekennzeichnet, dass**
e) zumindest eine der Leiterbahnen (21) bereichsweise auf einer Brillenscheiben-Innenseite (10) und bereichsweise auf einer Brillenscheiben-Außenseite (9) verläuft.

2. Brillenscheibe nach Anspruch 1, **dadurch gekennzeichnet, dass** die Heizzonen nebeneinander angeordnet sind.

3. Brillenscheibe nach Anspruch 1 oder 2, **dadurch gekennzeichnet, dass** die Leiterbahnen (21) und/oder Kontaktmittel (16, 17, 18, 19) aufgedruckt sind.

4. Brillenscheibe nach einem der vorherigen Ansprüche, **gekennzeichnet durch** eine transparente, wasserabsorbierende, innere Antibeschlag-Überbeschichtung (13).

5. Brillenscheibe nach einem der vorherigen Ansprüche, **gekennzeichnet durch** mindestens eine transparente, harte Schicht (14), die den Grundkörper (11) zumindest bereichsweise bedeckt.

6. Brillenscheibe nach einem der vorherigen Ansprüche, **dadurch gekennzeichnet, dass** der Grundkörper (11) zumindest bereichsweise gekrümmt ist.

7. Brillenscheibe nach einem der vorherigen Ansprüche, **dadurch gekennzeichnet, dass** bei der mindestens einen bereichsweise auf der Brillenscheiben-Innenseite (10) und bereichsweise auf der Brillenscheiben-Außenseite (9) verlaufenden Leiterbahn (21) ein Seitenwechsel (27) zwischen der Brillenscheiben-Innenseite (10) und Brillenscheiben-Außenseite (9) vorliegt.

8. Brillenscheibe nach Anspruch 7, **dadurch gekennzeichnet, dass** bei jedem Seitenwechsel (27) die jeweilige Leiterbahn (21) außen um den Grundkörper (11) herumläuft.

9. Brille mit mindestens einer Brillenscheibe (1) nach einem der vorherigen Ansprüche.

## Claims

1. Goggle lens
a) having a base body (11),
b) having at least one transparent, electrically conductive coating (12), which covers the base body (11) at least in areas,
c) having electrical contact means (16, 17, 18, 19), which
i) cover the at least one coating (12) in areas,
ii) are electrically connected to the at least one coating (12), and
iii) co-form at least two electrical heating zones, which are actuatable independently of one another, and
d) having electrical conductor tracks (21), which are electrically connected to
the contact means (16, 17, 18, 19) for applying current thereto, **characterised in that**
e) at least one of the conductor tracks (21) runs in some areas on a goggle lens inner side (10) and in some areas on a goggle lens outer side (9).

2. Goggle lens according to claim 1, **characterised in that** the heating zones are arranged next to one another.

3. Goggle lens according to claim 1 or 2, **characterised in that** the conductor tracks (21) and/or contact means (16, 17, 18, 19) are printed.

4. Goggle lens according to one of the preceding claims, **characterised by** a transparent, water-absorbing, inner anti-fog over coating (13).

5. Goggle lens according to one of the preceding claims, **characterised by** at least one transparent, hard layer (14), which covers the base body (14) at least in areas.

6. Goggle lens according to one of the preceding claims, **characterised in that** the base body (11) is curved at least in areas.

7. Goggle lens according to one of the preceding claims, **characterised in that** there is side change (27) between the goggle lens inner side (10) and the goggle lens outer side (9) at the at least one conductor track (21) running in some areas on the goggle lens inner side (10) and in some areas on the goggle lens outer side (9).

8. Goggle lens according to claim 7, **characterised in that** at each side change (27) the respective conductor track (21) runs around an outside of the base body (11).

9. Goggle having at least one goggle lens (1) according to one of the preceding claims.

## Revendications

1. Verre de lunettes
a) comportant un corps de base (11),
b) comportant un revêtement (12) transparent électriquement conducteur qui recouvre le corps de base (11) au moins par sections,
c) comportant des moyens de contact (16, 17, 18, 19) électriques, qui
i) recouvrent l'au moins un revêtement (12) par sections,
ii) sont en liaison électrique avec l'au moins un revêtement (12), et
iii) co-forment au moins deux zones chauffantes électriques qui peuvent être actionnées indépendamment l'une de l'autre, et
d) comportant des pistes conductrices (21) électriques, qui sont reliées électriquement aux moyens de contact (16, 17, 18, 19) pour appliquer un courant à ceux-ci,
**caractérisé en ce que**
e) au moins une des pistes conductrices (21) s'étend par sections sur un côté intérieur de verre de lunettes (10) et par sections sur un côté extérieur de verre de lunettes (9).

2. Verre de lunettes selon la revendication 1, **caractérisé en ce que** les zones chauffantes sont disposées côte à côte.

3. Verre de lunettes selon la revendication 1 ou 2, **caractérisé en ce que** les pistes conductrices (21) et/ou les moyens de contact (16, 17, 18, 19) sont imprimés.

4. Verre de lunettes selon l'une des revendications précédentes, **caractérisé par** une surcouche (13) intérieure antibuée, transparente et absorbant l'eau.

5. Verre de lunettes selon l'une des revendications précédentes, **caractérisé par** au moins une couche (14) dure transparente qui recouvre le corps principal (11) au moins par sections.

6. Verre de lunettes selon l'une des revendications précédentes, **caractérisé en ce que** le corps de base (11) est courbé au moins par sections.

7. Verre de lunettes selon l'une des revendications précédentes, **caractérisée en ce que**, pour l'au moins une piste conductrice (21) s'étendant par sections sur le côté intérieur de la verre de lunettes (10) et par sections sur le côté extérieur de verre de lunettes (9), il y a un changement de côté (27) entre le côté intérieur de verre de lunettes (10) et le côté extérieur de verre de lunettes (9).

8. Verre de lunettes selon la revendication 7, **caractérisé en ce que**, à chaque changement de côté (27), la piste conductrice (21) respective contourne le corps de base (11) par un extérieur.

9. Lunettes comportant au moins un verre de lunettes (1) selon l'une des revendications précédentes.
